# EUROPEAN PATENT SPECIFICATION

(11) **EP 1 129 779 B1**
(45) Date of publication and mention of the grant of the patent: **25.05.2005**
(21) Application number: 01104527.5
(22) Date of filing: 02.03.2001
(51) Int. Cl.: B01L 3/14, B04B 7/08, B04B 5/04, G01N 33/49

(54) **Container for centrifugation**
Zentrifugationsbehälter
Récipient pour centrifugation

(30) Priority: 02.03.2000 JP 2000107349
(43) Date of publication of application: 05.09.2001
(73) Proprietor: ARKRAY, Inc., Kyoto-shi, Kyoto 601-8045 (JP)
(72) Inventor: Kitamura, Shigeru, Arkray, Inc., Minami-ku, Kyoto-shi, Kyoto 601-8045 (JP); Matsuda, Takeshi, Arkray, Inc., Minami-ku, Kyoto-shi, Kyoto 601-8045 (JP)
(74) Representative: HOFFMANN - EITLE

(56) References cited:
- WO-A-90/15333
- US-A- 4 458 812
- US-A- 4 798 577

## Description

### BACKGROUND OF THE INVENTION

### 1. Field of the Invention

The present invention relates to a container for centrifugation. More specifically, the present invention relates to a container which preferably is used to separate a small amount of specimen by centrifugation.

### 2. Background Art Relating to the Invention

Generally, in blood analysis, a blood serum and hemocytes are centrifuged for taking out the blood serum. The blood serum is then allowed to react with a reagent for measuring the degree of color change in the reagent. In addition to blood analysis, a centrifugal separator is used widely for taking out a specific component from a fluid containing plural components with different specific gravities.

Most of the conventional centrifugal separators are large apparatuses comprising a rotation device to be rotated by a motor, and an even number of centrifugal tubes with one end closed. However, in addition to the necessity of a dummy for balancing, such a large apparatus involves the following problems.

First, the upper layer component in the centrifugal tubes cannot be taken out until the rotating member stops.

Second, when automatically collecting the upper layer component in the centrifugal tubes by a collecting tool of an analysis device, such as a absorption pipet, the stopping position of the centrifugal tubes should be detected for moving the collecting tool according to the detection signal, or the centrifugal tubes should be forcibly stopped at the position of the collecting tool. In either case, a complicated controlling circuit is required.

Third, since the centrifugal separator itself is large, an analysis device storing the centrifugal separator cannot be provided in a small size.

In order to solve the problems, a disc-like disposable small container for centrifugation has been proposed in, for example, JP-A-62-273065 (the term "JP-A" means an unexamined published Japanese patent application) by providing a centrifugation method capable of taking out a low specific gravity component needed for the analysis in a short time, automatically collecting the upper layer component by a collecting tool of an analysis device without the need of a complicated controlling circuit, and achieving a smaller size of the centrifugal separator.

Although an apparatus using the above-mentioned small centrifugation container provides excellent effects, a problem arises when the specimen is provided in a small amount, particularly when the separated low-specific-gravity component has viscosity (such as blood serum, blood plasma, or the like).

The above-noted problem arises because the low-specific-gravity component is adhered on an inner wall, of the disc-like container's dam, by centrifugal force. Thus, when the low-specific-gravity component is provided in a small amount with a high viscosity, it remains stuck on the inner wall even after the rotation stops. And the low-specific-gravity component does not easily drop into the inside of the container (i.e., the central room for suctioning the specimen) due to the component's viscosity. Because the component does not easily drop, it cannot easily be collected by a suction pipette, resulting in prolongation of the analysis time.

### SUMMARY OF THE INVENTION

Accordingly, an object of the present invention is to provide a container from which a component needed for analysis can be taken out in a short time, even if the component is of a small amount because the specimen is of a small amount.

This and other objects of the present invention have been attained by a container for centrifugation, comprising:
a central room including a rotation center;
a through hole opened to the upper part of the central room;
an outer wall surrounding the central room;
a ringed dam, not higher than the outer wall, surrounding the central room, wherein the ringed dam includes a surface that faces the central room; and
a peripheral room disposed between the dam and the outer wall,
wherein plural capillary phenomenon-inducing structures are formed on the surface of the dam facing the central room,
wherein the container is disc-like,
wherein the container has a rotation axis, and
wherein the container is capable of elongating outwardly from the rotation axis in the radial direction, and is capable of rotating around the rotation axis for separating a composite fluid into a low-specific-gravity component and a high-specific-gravity component by centrifugal force.

Furthermore, this and other objects of the present invention have been attained by a method for separating a composite fluid into a low-specific-gravity component and a high-specific-gravity component by centrifugal force, comprising:
charging the composite fluid into the central room of the above container; and
rotating the container.

### BRIEF EXPLANATION OF THE DRAWINGS

The above and other objects and advantages of the present invention will become more apparent by describing in detail preferred exemplary embodiments thereof with reference to the accompanying drawings, wherein like reference numerals designate like or corresponding parts throughout the several views, and wherein:
Fig. 1 is a perspective view of a container for centrifugation according to the present invention;
Fig. 2 is a cross-sectional view showing the state of the container for centrifugation of Fig. 1 with the cover detached;
Fig. 3 is a plan view of the container for centrifugation in the state of Fig. 2, as observed from above;
Fig. 4 is a cross-sectional view taken on the line IV-IV of Fig. 3; and
Fig. 5 is a plan view showing another embodiment of a container for centrifugation according to the present invention.

### DETAILED DESCRIPTION OF THE INVENTION

According to the present invention, a ringed dam not higher than an outer wall is provided inside the container for centrifugation. When the container is rotated, about its center of rotation, with a mixture containing at least two components, i.e., a high-specific-gravity component and a low-specific-gravity component, the high-specific-gravity component moves by centrifugal force over the dam, so as to be clashed against the outer wall, faster than does the low-specific-gravity component. Thereafter, the high-specific-gravity component is precipitated between the dam and the outer wall upon a decrease of the rotational frequency. In contrast, since the low-specific-gravity component is in the upper layer, it moves over the dam so as to flow backward toward the rotation center. Therefore, the centrifuged low-specific-gravity component can be gathered at the rotation center, whereas the high-specific-gravity component is blocked by the dam and is collected between the dam and the outer wall. Hence, even when a collecting tool, such as a pipette chip, is inserted in the low-specific-gravity component at the rotation center, it is not re-mixed with the high-specific-gravity component. As a result, the low-specific-gravity component can be collected without the need to await stoppage of the rotating container. Besides, a complicated controlling circuit is not required for moving the collecting tool. Furthermore, even when the obtained low-specific-gravity component is of a small amount that is adhered on the inner wall of the dam, it soon drops to the bottom of the dam due to the plural capillary phenomenon-inducing structures formed on the surface of the dam facing the central room. Thus, collection of the low-specific-gravity component is facilitated.

By detecting the rotational frequency of the circular motion for generating the centrifugal force during the centrifugation method according to the present invention, and by soaking the collecting tool into the low-specific-gravity component (for collection thereof) by moving the collecting tool along the rotation axis when the rotational frequency is a predetermined value, the collecting operation can be carried out a short time after the start of centrifugation.

According to the container for centrifugation of the present invention, since the container itself has a rotating member shape, the centrifugal force can be generated by rotating the container with the center thereof coincident with the centrifugal axis. Therefore, a dummy container can be eliminated. Moreover, since the above-mentioned dam is provided, the mixture moves over the dam during the centrifugation so as to clash against the outer wall, and the high-specific-gravity component is precipitated between the dam and the outer wall upon a decrease of the rotational frequency. In contrast, the low-specific-gravity component moves over the dam backward so as to be gathered at the rotation center. Therefore, the low-specific-gravity component and the high-specific-gravity component are not re-mixed after the centrifugation. Moreover, since the low-specific-gravity component is gathered at the rotation center position, the low-specific-gravity component can be collected from the rotation center.

Furthermore, the dam is provided with plural capillary phenomenon-inducing structures formed on the inside surface thereof, that is, the surface facing the central room surrounded by the ringed dam. The capillary phenomenon-inducing structures, for promoting movement of the specimen adhered on the wall surface of the dam by the capillary tube phenomenon, can be provided in either of two ways including an engraved groove and an upright rib. The number of capillary phenomenon-inducing structures is at least two and, preferably, is as large as possible.

In the container for centrifugation according to the present invention, a cover is optionally provided. If the cover is not provided, the fluid can be prevented from spilling, during the centrifugation, by making the outer wall of the container sufficiently higher than the dam. A through hole, for collecting a specimen in the central room, preferably is provided above the rotation center. Therefore, the "through hole" in the present invention can be the absence of a cover.

When the cover is made of a rigid material, it is preferable that the dam has a height such that the upper end thereof does not contact with the cover. A gap between the upper end of the dam and the cover can serve as the channel for outwardly moving a high-specific-gravity component. Alternatively, such a gap can serve for moving a low-specific-gravity component toward the rotation center.

When the cover is made of an elastic material, it is preferable that the cover is bonded with the outer wall of the container, and that the dam has a height so that the upper end thereof contacts with the cover. Since the cover is bonded with the outer wall of the container, when a centrifugal force is generated, the cover is deformed elastically so as to be bulged upward, thereby forming a gap with respect to the upper end of the dam. Therefore, a high-specific-gravity component can move over the dam through the gap. In contrast, since the cover regains its original shape upon a decline of the centrifugal force, it contacts the dam, and thus more surely prevents re-mixing of the high-specific-gravity component that is outside the dam, and the low-specific-gravity component that is inside the dam.

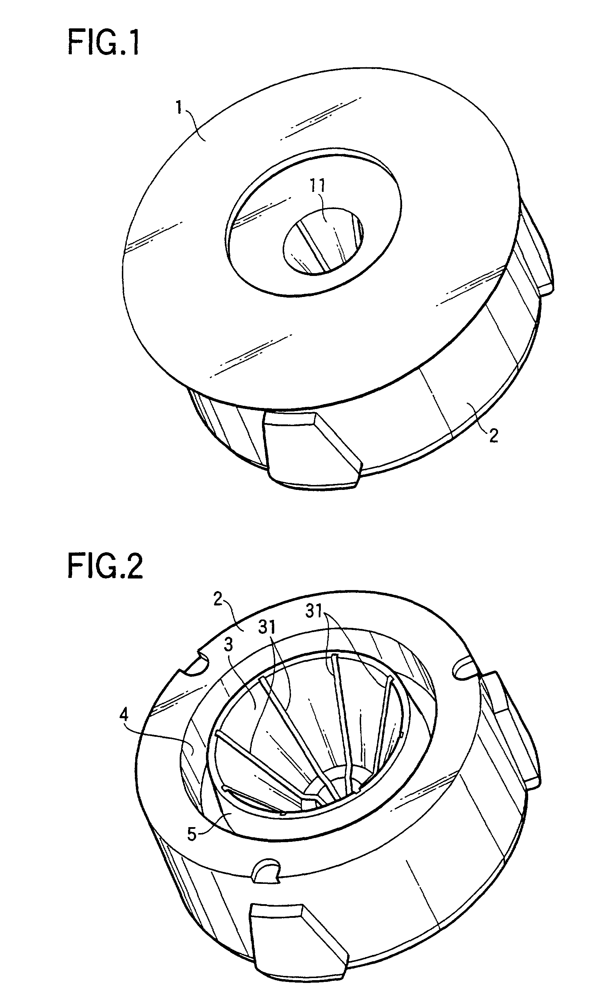

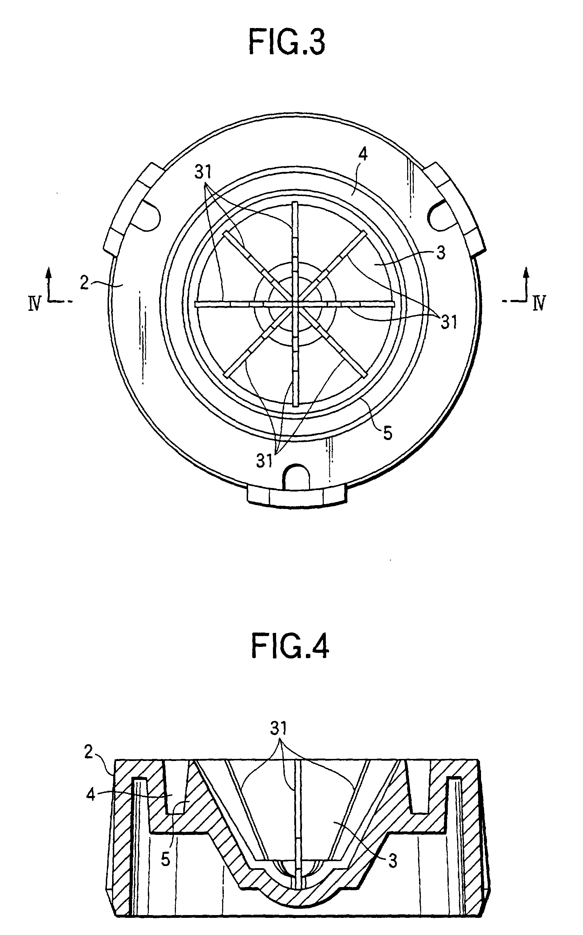

According to the present invention, an embodiment with a cover made of a rigid material will be explained with reference to the drawings. Fig. 1 is a perspective view showing a container for centrifugation according to a first embodiment. Fig. 2 shows the container with the cover detached. Fig. 3 shows the state with the cover detached, and as observed from above. Fig. 4 is a cross-sectional view taken on the line IV-IV in Fig. 3. The container for centrifugation according to the present invention comprises a body 2 opened on its top, having a rotating member shape, and a cover 1 for closing the opening of the body 2. As described above, the cover 1 is optionally provided in the present invention. The material of the body 2 and the cover 1 is a resin capable of providing a suitable rigidity, such as a polystyrene, an ABS resin, a polycarbonate, a polypropylene, and a polyethylene. Therefore, the container for centrifugation can be produced easily by a known technique, such as injection molding, cut processing, or the like. Inside the body 2, a central room 3 is formed by providing a columnar dam 5 in the central part, including the rotation center, and a peripheral room 4 is formed around the same. The bottom of the peripheral room 4 is a flat surface. The cover 1 has a flat disc-like shape, except for a through hole 11 for taking in or out a specimen provided at the center.

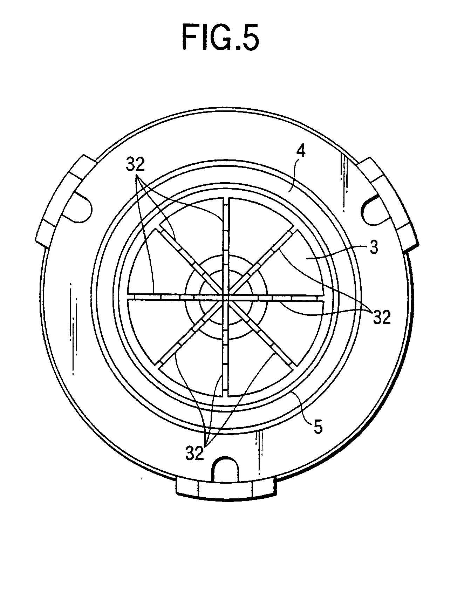

As is shown in Fig. 3, eight grooves 31 are engraved in the side of the dam 5 as capillary phenomenon-inducing structures. In Fig. 5, eight ribs 32 are protrudingly provided on the side of the dam 5 as capillary phenomenon-inducing structures.

The operation for the centrifugation of a liquid mixture containing two components with different specific gravities, such as blood containing blood serum (a low-specific-gravity component) and hemocytes (a high-specific-gravity component), using the container for centrifugation according to the present invention, and the phenomenon generated in the container for centrifugation at the time, will be described.

First, the tip end of a syringe or the like is inserted from the through hole 11 into the container for centrifugation, with the cover 1 placed on the body 2, for charging the blood into the central room 3. The amount of the charge is such that the liquid level is higher than the dam 5, but not contacting with the inner surface of the cover 1. For rotating the container for centrifugation, the container for centrifugation is fixed on a rotating device. The blood can be charged after fixing the container for centrifugation on the rotating device. By rotating the container for centrifugation in this state, by driving the rotating device, the blood is separated into blood corpuscle located outside the peripheral room 4, and blood serum and blood plasma located inside the dam 5 (i.e., in the central room 3).

After passage of a predetermined time with a predetermined rotational frequency, the driving force of the rotating device either is stopped so that rotation continues by inertia, or is gradually lowered so that the output of the rotating device is lowered gradually. Since the centrifugal force is reduced according to the decline of the rotational frequency, the blood corpuscle is gathered at the bottom of the peripheral room 4. Also, since the height of the dam 5 serves as an obstacle, the blood corpuscle cannot move over the upper surface of the dam 5 so as to enter the central room 3. Therefore, only the blood serum and the blood plasma are suctioned by inserting, through the through hole 11, a suction pipette into the container for centrifugation. Moreover, since the through hole 11 always is located at the rotation center during the rotation of the container for centrifugation, the blood serum and the blood plasma can be suctioned by inserting the suction pipette into the container for centrifugation without awaiting for a complete stoppage of the container for centrifugation. Hence the analysis can be started a short time after the centrifugation.

When the absolute specimen amount is small, or the centrifugal force torque is weak for some reason, the centrifuged blood serum and blood plasma after the centrifugation may be of a small amount. Since the blood serum and the blood plasma have viscosity, when their amount is little, they hardly drop from the wall surface by their own weight. However, since a capillary phenomenon-inducing structure (groove 31 or rib 32) is formed in the dam 5 of the container for centrifugation according to the present invention, even a specimen of a small amount can be collected in a short time.

As discussed above, according to the container for centrifugation of the present invention, a low-specific-gravity component is gathered at the rotation center so that it can be collected without awaiting stoppage of the rotating member. Also, the low-specific-gravity component needed for analysis can be collected in a short time, and can be collected immediately after finish of the rotation, even in the case of a specimen of a small amount. Furthermore, a centrifugal separator can be provided in a small size.

The present invention will be explained, in detail, by the following examples; however, the present invention is not limited thereto.

### Example 1

This is an experimental example of centrifugation of blood, using a container for centrifugation according to the present invention having a groove as the capillary phenomenon-inducing structure. In the container for centrifugation shown in Figs. 1 to 4, the inner diameter of the body 2 was set at 14 mm, the depth of the peripheral room 4 at 4 mm, the height of the dam 5 at 2 mm, and the outer diameter of the dam 5 at 6 mm. Eight grooves 31 (0.3 mm width, and 0.5 mm depth) were formed on the inner wall of the dam 5 that faces toward the rotation axis center. As a comparative example, a container for centrifugation with the same structure as set forth above, except for not having a groove, was prepared.

Blood of 250 µl, having a hematocrit value of 46, was charged into the central room 3 of both containers. The lids 1 were placed thereon, and the containers were rotated in the atmosphere at an ordinary temperature by 10,000 rpm for 3 minutes. For each container, the output of the motor to serve as the rotation driving source at the time was 1.3 V, and the centrifugal force at the time was calculated to be 783 G. Then, for each container, the motor output was reduced by a 0.1 V/second rate, and by a 0.05 V/second rate from when it was lowered to 0.3 V until the containers for centrifugation were finally stopped. As a result, most of the blood corpuscle was gathered in the peripheral room 4. By inserting a pipette from the through hole 11, the blood serum gathered inside the central room 3 was collected. The operation was repeated 5 times.

As a result, in the case of the container with the grooves, the blood serum could be collected immediately after stoppage of the rotation in each of the 5 times so as to collect the blood serum of 85 to 90 µl. However, in the case of the groove-free container, four times the blood serum could not be collected immediately after stoppage of the rotation, and once only 30 µl was collected immediately after stoppage of the rotation.

In generally used containers, about 500 µl of blood is required, whereas according to the present invention, blood of 250 µl or less could be separated.

### Example 2

This is an experimental example of centrifugation of blood, using a container for centrifugation according to the present invention having a rib as the capillary phenomenon-inducing structure. A container for centrifugation was prepared with the same size as the container in Example 1 except that eight ribs (0.3 mm width, and 0.5 mm height) were provided instead of the grooves. As a comparative example, a container for centrifugation with the same structure, except for not having a rib, was prepared.

Blood of 250 µl, having a hematocrit value of 46, was charged into the central room 3 of each container. The separating operation was executed in the same manner as in Example 1.

As a result, in the case of the container with the ribs, the blood serum could be collected immediately after stoppage of the rotation in each of the 5 times so as to collect blood serum of 85 to 90 µl. However, in the case of the rib-free container, four times the blood serum could not be collected immediately after stoppage of the rotation, and once only 30 µl was collected immediately after stoppage of the rotation.

In generally used containers, about 500 µl of blood is required, whereas according to the present invention, blood of 250 µl or less could be separated.

While the invention has been described in detail and with reference to specific embodiments thereof, it will be apparent to one skill in the art that various changes and modifications can be made therein without departing from the scope thereof, as defined by the claims.

## Claims

1. A container for centrifugation, comprising:
a central room (3) including a rotation center;
a through hole (11) opened to the upper part of the central room;
an outer wall surrounding the central room;
a ringed dam (5), not higher than the outer wall, surrounding the central room, wherein the ringed dam includes a surface that faces the central room; and
a peripheral room disposed between the dam and the outer wall,
wherein the container is disc-like,
wherein the container has a rotation axis, and
wherein the container is capable of elongating outwardly from the rotation axis in the radial direction, and is capable of rotating around the rotation axis for separating a composite fluid into a low-specific-gravity component and a high-specific-gravity component by centrifugal force,
**characterised in that** plural capillary phenomenon-inducing structures (31,32) are formed on the surface of the dam facing the central room.

2. The container according to claim 1, wherein the capillary phenomenon-inducing structures are vertical grooves engraved on the surface of the ringed dam.

3. The container according to claim 1, wherein the capillary phenomenon-inducing structures are ribs disposed on the surface of the ringed dam.

4. A method for separating a composite fluid into a low-specific-gravity component and a high-specific-gravity component by centrifugal force, comprising:
charging the composite fluid into the central room of the container according to claim 1; and
rotating the container.

5. The method according to claim 4, wherein the composite fluid is blood.

## Patentansprüche

1. Zentrifugierbehälter, umfassend:
einen zentralen Raum (3) einschließlich eines Rotationsmittelpunkts;
ein durchgehendes Loch (11), das zum oberen Teil des zentralen Raums geöffnet ist;
eine den zentralen Raum umgebende Wand;
einen ringförmigen Damm (5), nicht höher als die äußere Wand, der den zentralen Raum umgibt, wobei der ringförmige Damm eine dem zentralen Raum gegenüberliegende Oberfläche aufweist; und
einen peripheren Raum, der zwischen dem Damm und der äußeren Wand angeordnet ist,
wobei der Behälter Scheiben-artig ist,
wobei der Behälter eine Rotationsachse aufweist, und
wobei der Behälter in der Lage ist, sich von der Rotationsachse radial auswärts zu verlängern, und in der Lage ist, um die Rotationsachse zu rotieren, um durch Zentrifugalkraft eine Mischflüssigkeit in Komponenten niedriger spezifischer Dichte und hoher spezifischer Dichte zu trennen,
**dadurch gekennzeichnet, dass**
mehrere Phänomen verursachende Kapillarstrukturen (31, 32) auf der Oberfläche des dem zentralen Raum gegenüberliegenden, ringförmigen Damms ausgebildet sind.

2. Der Behälter nach Anspruch 1, wobei die Phänomen verursachenden Kapillarstrukturen vertikale Rillen sind, die auf der Oberfläche des ringförmigen Damms eingraviert sind

3. Der Behälter nach Anspruch 1, wobei die Phänomen verursachenden Kapillarstrukturen auf der Oberfläche des ringförmigen Damms angeordnete Rippen sind.

4. Verfahren zum Trennen einer Mischflüssigkeit durch Zentrifugalkraft in eine Komponente niedriger spezifischer Dichte und hoher spezifischer Dichte, die folgenden Schritte umfassend:
Einlass der Mischflüssigkeit in den zentralen Raum des Behälters entsprechend Anspruch 1; und
Drehen des Behälters.

5. Verfahren nach Anspruch 4, wobei die Mischflüssigkeit Blut ist.

## Revendications

1. Récipient pour centrifugation, comprenant :
une pièce centrale (3) comprenant un centre de rotation ;
un trou de passage (11) ouvert sur la partie supérieure de la pièce centrale ;
une paroi extérieure entourant la pièce centrale ;
une retenue annulaire (5), pas plus élevée que la paroi extérieure, entourant la pièce centrale, dans lequel la retenue annulaire comprend une surface qui fait face à la pièce centrale ; et
une pièce périphérique disposée entre la retenue et la paroi extérieure,
dans lequel le récipient a la forme d'un disque,
dans lequel le récipient a un axe de rotation, et
dans lequel le récipient est capable de s'allonger vers l'extérieur depuis l'axe de rotation dans la direction radiale, et est capable de tourner autour de l'axe de rotation pour séparer un fluide composite en un composant à gravité spécifique basse et en un composant à gravité spécifique élevée par une force centrifuge,
**caractérisé en ce qu'**une pluralité de structures d'induction de phénomène capillaire (31, 32) est formée sur la surface de la retenue faisant face à la pièce centrale.

2. Récipient selon la revendication 1, dans lequel les structures d'induction de phénomène capillaire sont des rainures verticales gravées sur la surface de la retenue annulaire.

3. Récipient selon la revendication 1, dans lequel les structures d'induction de phénomène capillaire sont des rainures disposées sur la surface de la retenue annulaire.

4. Procédé destiné à séparer un fluide composite en un composant à gravité spécifique basse et en un composant à gravité spécifique élevée par une force centrifuge, comprenant les étapes consistant à :
charger le fluide composite dans la pièce centrale du récipient selon la revendication 1 ; et
faire tourner le récipient.

5. Procédé selon la revendication 4, dans lequel le fluide composite est du sang.
